Europäisches Patentamt

European Patent Office

Office européen des brevets

(19)

(11) Publication number: **0 475 148 A1**

## EUROPEAN PATENT APPLICATION

(12)

(21) Application number: **91114074.7**

(22) Date of filing: **22.08.91**

(51) Int. Cl.5: **A61K 31/215**, //(A61K31/215, 31:19)

(30) Priority: **23.08.90 US 571685**

(43) Date of publication of application:
**18.03.92 Bulletin 92/12**

(84) Designated Contracting States:
**AT BE CH DE DK ES FR GB GR IT LI LU NL SE**

(71) Applicant: **E.R. SOUIBB & SONS, INC.**
**P.O.Box 4000**
**Princeton New Jersey 08543-4000(US)**

(72) Inventor: **Pan, Henry Y. Dr.**
**7 Huntington Drive**
**Princeton Junction, New Jersey 08550(US)**

(74) Representative: **Vossius & Partner**
**Siebertstrasse 4 P.O. Box 86 07 67**
**W-8000 München 86(DE)**

(54) **Pravastatin alone or in combination with a fibric acid derivative for preventing onset of or treating type III hyperlipoproteinemia.**

(57) The use of pravastatin alone or in combination with a fibric acid derivative such as fenofibrate, gemfibrozil or bezafibrate for the preparation of a pharmaceutical composition useful in preventing or treating Type III hyperlipoproteinemia is described.

EP 0 475 148 A1

The present invention relates to the use of an HGM CoA reductase inhibitor, which is pravastatin, alone or in combination with a derivative of fibric acid such as fenofibrate or gemfibrozil for the preparation of a pharmaceutical composition useful for preventing the onset of or treating Type III hyperlipoproteinemia.

As indicated in the Merck Manual, 15th Ed., p. 1008, Type III hyperlipoproteinemia, also referred to as dysbetalipoproteinemia, is a familial disorder characterized by the accumulation in blood serum of a beta migrating very low-density lipoprotein (VLDL), rich in triglycerides and cholesterol, associated with a marked predisposition to severe mature atherosclerosis. It is often associated with abnormalities of apolipoprotein E and defective conversion and removal of VLDL from the plasma.

East, C.A. et al, "Treatment of type 3 hyperlipoproteinemia with mevinolin," Metab. Clin. Exp. (USA), 1986, 35/2 (97-98), disclose that mevinolin markedly lowered both VLDL remnants and LDL in a patient with type 3 hyperlipoproteinemia.

Yoshino et al, "Effects of CS-514 Pravastatin on VLDL-Triglyceride Kinetics in Rats," Atherosclerosis 73 (2-3), 1988, 191-196, discloses that CS-514, an inhibitor of cholesterol biosynthesis can suppress VLDL-triglyceride secretion in rats and that this effect can be modified by dietary manipulation.

Pravastatin, also referred to as CS-514, an HMG CoA reductase inhibitor, is disclosed in U.S. Patent No. 4,346,227.

Sirtori, C.R. et al, "Effects of Fibrates on Serum Lipids and Atherosclerosis," Pharmac. Ther., Vol. 37, pp. 167 to 191, 1988 (G.B.), discloses that various fibrates, namely, bezafibrate, ciprofibrate, fenofibrate, gemfibrozil and clofibrate, possess lipid-lowering effects and reduce serum cholesterol and triglyceride levels.

The use of combinations of various types of cholesterol lowering drugs have been suggested in the literature. For example, Brown, W.V., "Potential Use of Fenofibrate and Other Fibric Acid Derivatives in the Clinic," Am. J. Med., Nov. 27, 1987, Vol. 83 (suppl 5B), indicates at page 85 that "in combination with other agents that lower low-density lipoprotein levels more specifically, such as the bile acid sequestrants and hydroxymethylglutaryl coenzyme A reductase inhibitors, fenofibrate may act to effect control of the triglycerides allowing management of those patients with disorders producing elevated very low-density lipoprotein and low-density lipoprotein levels."

Weisweiler, P., "Simvastatin and Bezafibrate Effects on Serum Lipoproteins and Lecithin Cholesterol Acyltransferase Activity in Familial Hypercholesterolemia," Eur. J. Clin. Pharmacol. 35(6), 1988, 579-584, indicates that "simvastatin was clearly more effective than bezafibrate in lowering LDL by enhancing its turnover, but bezafibrate had specific effects on VLDL and HDL that might be favorable in combined treatment regiments."

East, C. et al, in a letter to the Editor N. Engl. J. Med., Vol. 318, No. 1, Jan. 7, 1988, pp. 47-48, disclose that 4 patients after cardiac transplant received lovastatin (2 of which also received gemfibrozil) and developed rhabdomyolysis.

Physicians' Desk Reference, 44th Ed., 1990, page 1413 reports that severe cases of rhabdomyolysis have been associated in patients receiving lovastatin in combination with either gemfibrozil or lipid-lowering doses of nicotinic acid. "Acute renal failure from rhabdomyolysis has been seen more commonly with the lovastatin-gemfibrozil combination, and has also been reported in transplant patients receiving lovastatin plus cyclosporine."

"Fulminant rhabdomyolysis has been seen as early as three weeks after initiation of combined therapy with gemfibrozil and lovastatin, but may be seen after several months. For these reasons, it is felt that, in most subjects who have had an unsatisfactory lipid response to either drug alone, the possible benefits of combined therapy with lovastatin and gemfibrozil do not outweigh the risks of severe myopathy, rhabdomyolysis, and acute renal failure. While it is not known whether this interaction occurs with fibrates other than gemfibrozil, myopathy and rhabdomyolysis have occasionally been associated with the use of other fibrates alone, including clofibrate. Therefore, the combined use of lovastatin with other fibrates should generally be avoided."

Illingworth, D.R.; Bacon, S., Am. J. Cardiol. (60, No. 12, 33G-42G, 1987) in their paper entitled "Hypolipidemic Effects of HMG CoA Reductase Inhibitors in Patients with Hypercholesterolemia," disclose the use of 3-hydroxy 3-methylglutaryl coenzyme A (HMG CoA) reductase inhibitors such as mevastatin (compactin), lovastatin (mevinolin), simvastatin (synvinolin) and pravastatin (CS-514, eptastatin or SQ-31000) in the treatment of familial hypercholesterolemia. "In patients who do not experience adequate control with HMG CoA reductase inhibitors combination therapy with cholestyramine, colestipol, neomycin, probucol, nicotinic acid, gemfibrozil, bezafibrate, fenofibrate, ciprofibrate or clofibrate may be initiated."

Illingworth, D.R., Clin. Chem. (34,No. 8, Suppl., B123-B132, 1988), in a paper entitled "Drug Therapy of Hypercholesterolemia," discloses that drugs available for treatment of hypercholesterolemia include cholestyramine and colestipol, nicotinic acid, HMG CoA reductase inhibitors such as lovastatin, mevastatin,

simvastatin, pravastatin and SRl62320; fibric acid derivatives such as clofibrate, gemfibrozil, fenofibrate, bezafibrate and ciprofibrate, probucol, neomycin, and d-thyroxin. "Combinations of 2 or 3 drugs with different mechanisms of action can be used in severe hypercholesterolemia. It is concluded that currently available drugs are capable of reducing plasma cholesterol sufficiently to alter the course of premature atherosclerosis in patients with severe hypercholesterolemia."

It is also known that Type III hyperlipoproteinemia has been treated with fibric acid derivatives, Bhatnagar et al, "Borderline hypercholesterolaemia: when to introduce drugs," Postgraduate Medical Journal (1989) 65, 543-552; Lussier et al, "Lipoprotein Composition Changes Induced by Fenofibrate in Dysbetalipoproteinemia Type III," Atherosclerosis, 78, No. 2-3, 167-182, 1989; Illingworth, D.R. "An Overview of Lipid-Lowering Drugs," Drugs 36 (Suppl.3):63-71 (1988) (clofibrate, nicotinic acid or lovastatin); Kuo et al, "Treatment of Type III Hyperlipoproteinemia with Gemfibrozil to Retard Progression of Coronary Artery Disease," Am. Heart J., 116, No. 1, Pt. 1, 85-90, 1988; Illingworth et al, "The hypolipodemic effects of lovastatin and clofibrate alone and in combination in patents with type III hyperlipoproteinemia," Metabolism (US) Apr. 1990, 39(4) p. 403-9.

The present invention relates to the use of pravastatin alone or in combination with a fibric acid derivative for the preparation of a pharmaceutical composition useful for preventing the onset of or treating Type III hyperlipoproteinemia, which may be administered systemically, such as orally or parenterally.

Pravastatin will be employed in a weight ratio to the fibric acid derivative of within the range of from about 0.001:1 to about 1000:1 and preferably from about 0.05:1 to about 100:1.

It has been found that the combination of pravastatin and the fibric acid derivative, which works by a mechanism other than inhibiting HMG CoA reductase, is a surprising and unique concept in treating Type III hyperlipoproteinemia in that it may provide additional anticholesterolemic effects over that which may be obtained using each of the components of the combination alone. Reduced levels of each of the pravastatin and the fibric acid derivative may be employed to achieve desired results, albeit with reduced side effects.

Surprisingly, the combination of pravastatin and the fibric acid derivative of the invention which is useful in treating Type III hyperlipoproteinemia is substantially safer than use of the combination of lovastatin and fibric acid derivatives.

The fibric acid derivatives suitable for use herein include, but are not limited to, fenofibrate, gemfibrozil, clofibrate, bezafibrate, ciprofibrate, clinofibrate and the like.

Preferred are combinations of pravastatin with fenofibrate, gemfibrozil or bezafibrate.

The pharmaceutical compositions prepared according to the invention may be administered to mammalian species, such as monkeys, dogs, cats, rats, humans, etc. and as such may be incorporated in a conventional systemic dosage form, such as a tablet, capsule, elixir or injectable. The above dosage forms will also include the necessary carrier material, excipient, lubricant, buffer, antibacterial, bulking agent (such as mannitol), anti-oxidants (ascorbic acid or sodium bisulfite) or the like. Oral dosage forms are preferred, although parenteral forms are quite satisfactory as well.

The dose administered must be carefully adjusted according to age, weight and condition of the patient, as well as the route of administration, dosage form and regimen and the desired result.

Thus, for oral administration, a satisfactory result may be obtained employing the pravastatin in dosages employed, for example, for lovastatin as indicated in the Physicians' Desk Reference, such as in an amount within the range of from about 1 to 2000 mg and preferably from about 4 to about 200 mg in combination with the fibric acid derivative in dosages normally for these cholesterol lowering drugs, for example, as indicated in the Physicians' Desk Reference, in the case of gemfibrizol, and clofibrate such as in an amount within the range of from about 2 mg to about 7500 mg and preferably from about 2 mg to about 4000 mg, with pravastatin and the fibric acid derivative being employed together in the same oral dosage form or in separate oral dosage forms taken at the same time.

A preferred oral dosage form, such as tablets or capsules, will contain the pravastatin in an amount of from about 0.1 to about 100 mg, preferably from about 5 to about 80 mg, and more preferably from about 10 to about 40 mg, and the fibric acid derivative in an amount of from about 2 to about 3000 mg, preferably from about 2 to about 2000 mg.

The composition described above may be administered in the dosage forms as described above in single or divided doses of one to four times daily. It may be advisable to start a patient on a low dose combination and work up gradually to a high dose combination.

Tablets of various sizes can be prepared, e.g., of about 2 to 2000 mg in total weight, containing one or both of the active substances in the ranges described above, with the remainder being a physiologically acceptable carrier of other materials according to accepted pharmaceutical practice. These tablets can, of course, be scored to provide for fractional doses. Gelatin capsules can be similarly formulated.

Liquid formulations can also be prepared by dissolving or suspending one or the combination of active substances in a conventional liquid vehicle acceptable for pharmaceutical administration so as to provide the desired dosage in one to four teaspoonsful.

Such dosage forms can be administered to the patient on a regimen of one to four doses per day.

According to another modification, in order to more finely regulate the dosage schedule, the active substances may be administered separately in individual dosage units at the same time or carefully coordinated times. Since blood levels are built up and maintained by a regulated schedule of administration, the same result is achieved by the simultaneous presence of the two substances. The respective substances can be individually formulated in separate unit dosage forms in a manner similar to that described above.

Fixed combinations of pravastatin and fibric acid derivative are more convenient and are preferred, especially in tablet or capsule form for oral administration.

In formulating the compositions, the active substances, in the amounts described above, are compounded according to accepted pharmaceutical practice with a physiologically acceptable vehicle, carrier, excipient, binder, preservative, stabilizer, flavor, etc., in the particular type of unit dosage form.

Illustrative of the adjuvants which may be incorporated in tablets are the following: a binder such as gum tragacanth, acacia, corn starch or gelatin; an excipient such as dicalcium phosphate or cellulose; a disintegrating agent such as corn starch, potato starch, alginic acid or the like; a lubricant such as stearic acid or magnesium stearate; a sweetening agent such as sucrose, aspartame, lactose or saccharin; a flavoring agent such as orange, peppermint, oil of wintergreen or cherry. When the dosage unit form is a capsule, it may contain in addition to materials of the above type a liquid carrier such as a fatty oil. Various other materials may be present as coatings or to otherwise modify the physical form of the dosage unit. For instance, tablets or capsules may be coated with shellac, sugar or both. A syrup of elixir may contain the active compound, water, alcohol or the like as the carrier, glycerol as solubilizer, sucrose as sweetening agent, methyl and propyl parabens as preservatives, a dye and a flavoring such as cherry or orange.

Some of the active substances described: above form commonly known, pharmaceutically acceptable salts such as alkali metal and other common basic salts or acid addition salts, etc. References to the base substances are therefore intended to include those common salts known to be substantially equivalent to the parent compound.

The formulations as described above will be administered for a prolonged period, that is, for 4 weeks to 6 months or longer, as long as the symptoms of Type III hyperlipoproteinemia continue. Sustained release forms of such formulations which may provide such amounts biweekly, weekly, monthly and the like may also be employed. A dosing period of at least one to two weeks are required to achieve minimal benefit.

The following Examples represent preferred embodiments of the present invention.

Example 1

A pravastatin formulation in the form of tablets having the following composition was prepared as described below.

| Ingredient | Parts by Weight |
|---|---|
| Pravastatin | 7 |
| Lactose | 67 |
| Microcrystalline cellulose | 20 |
| Croscarmellose sodium | 2 |
| Magnesium stearate | 1 |
| Magnesium oxide | 3 |

Pravastatin, magnesium oxide and a fraction (30%) of the lactose were mixed together for 2 to 10 minutes employing a suitable mixer. The resulting mixture was passed through a #12 to #40 mesh size screen. Microcrystalline cellulose, croscarmellose sodium and the remaining lactose were added and the mixture was mixed for 2 to 10 minutes. Thereafter, magnesium stearate was added and mixing was continued for 1 to 3 minutes.

The resulting homogeneous mixture was then compressed into tablets each containing 5 mg, 10 mg, 20 mg or 40 mg pravastatin which may be used in preventing or treating Type III hyperlipoproteinemia.

Examples 2

Pravastatin tablets are prepared employing conventional pharmaceutical techniques containing 20 mg pravastatin and inert ingredients employed in lovastatin tablets, namely cellulose, color, lactose, magnesium stearate and starch and butylated hydroxyanisole as a preservative as described in the 1990 PDR.

The pravastatin tablets may be employed to prevent or treat Type III hyperlipoproteinemia in accordance with the present invention.

Example 3

A pravastatin tablet formulation is prepared as described in Example 1.

Fenofibrate tablets containing 250 mg fenofibrate are prepared employing conventional procedures containing the following additional ingredients: corn starch, ethyl cellulose, glycerin, hydroxypropyl cellulose, hydroxypropylmethyl cellulose 2910, iron oxide, lactose, magnesium stearate, microcrystalline cellulose, polysorbate 80, talc and titanium dioxide.

The pravastatin tablets and fenofibrate tablets may be administered as a combination in accordance with the teachings of the present invention to prevent or treat Type III hyperlipoproteinemia. In addition, the pravastatin and fenofibrate tablets may be ground up into powders and used together in a single capsule.

Examples 4

Pravastatin tablets are prepared as described in Example 2.

The pravastatin tablets may be employed in combination with gemfibrozil tablets containing 300 mg gemfibrozil and inactive ingredients as described in the 1990 PDR, in separate or combined dosage forms to treat Type III hyperlipoproteinemia in accordance with the present invention.

Examples 5

Pravastatin tablets, described in Examples 1 and 2, respectively, may be employed in combination with clofibrate tablets containing 500 mg clofibrate and inactive ingredients as described in the 1990 PDR. The pravastatin and clofibrate may be employed in separate dosage forms or combined in a single capsule form to treat Type III hyperlipoproteinemia in accordance with the present invention.

Examples 6 to 8

Combinations of pravastatin and ciprofibrate, bezafibrate and clinofibrate may also be prepared in a manner described hereinbefore in Example 3 to 5 for use in treating Type III hyperlipoproteinemia.

**Claims**

1. Use of pravastatin for the preparation of a pharmaceutical composition useful in preventing or treating Type III hyperlipoproteinemia.

2. The use as defined in claim 1, wherein the pharmaceutical composition prepared is such that said pravastatin is administered in single or divided doses of from about 1 to about 2000 mg/one to four times daily.

3. The use as defined in Claim 1, wherein the pharmaceutical composition prepared is such that the pravastatin is administered in single or divided doses of from about 4 to about 200 mg/one to four times daily.

4. Use of pravastatin for the preparation of a pharmaceutical composition useful in preventing or treating Type III hyperlipoproteinemia, which additionally comprises a fibric acid derivative.

5. The use as defined in Claim 4, wherein said fibric acid derivative is gemfibrozil, fenofibrate, clofibrate, bezafibrate, ciprofibrate or clinofibrate.

6. The use as defined in Claim 4, wherein said fibric acid derivative is fenofibrate, gemfibrozil or bezafibrate.

7.  The use as defined in Claim 4, wherein pravastatin is present in the pharmaceutical composition in a weight ratio to the fibric acid derivative of within the range of from about 0.001:1 to about 1000:1.

8.  The use as defined in Claim 4, wherein the fibric acid derivative is fenofibrate.

9.  The use as defined in Claim 4, wherein the fibric acid derivative is gemfibrozil.

10. The use as defined in Claim 4, wherein the fibric acid derivative is bezafibrate.

**Claims for the following Contracting State: ES**

1.  A method for the preparation of a pharmaceutical composition useful in preventing or treating Type III hyperlipoproteinemia, which comprises an effective amount of pravastatin and a pharmaceutical acceptable carrier.

2.  The method as defined in Claim 1, wherein the pharmaceutical composition prepared is such that said pravastatin is administered in single or divided doses of from about 1 to about 2000 mg/one to four times daily.

3.  The method as defined in Claim 1, wherein the pharmaceutical composition prepared is such that the pravastatin is administered in single or divided doses of from about 4 to about 200 mg/one to four times daily.

4.  A method for the preparation of a pharmaceutical composition useful for preventing or treating Type III hyperlipoproteinemia, which comprises a therapeutically effective amount of pravastatin in combination with a fibric acid derivative.

5.  The method as defined in Claim 4, wherein said fibric acid derivative is gemfibrozil, fenofibrate, clofibrate, bezafibrate, ciprofibrate or clinofibrate.

6.  The method as defined in Claim 4, wherein said fibric acid derivative is fenofibrate, gemfibrozil or bezafibrate.

7.  The method as defined in Claim 4, wherein pravastatin is present in the pharmaceutical preparation in a weight ratio to the fibric acid derivative of within the range of from about 0.001:1 to about 1000:1.

8.  The method as defined in Claim 4, wherein the fibric acid derivative is fenofibrate.

9.  The method as defined in Claim 4, wherein the fibric acid derivative is gemfibrozil.

10. The method as defined in Claim 4, wherein the fibric acid derivative is bezafibrate.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,D | THE AMERICAN JOURNAL OF CARDIOLOGY, vol. 60, no. 12, 30th October 1987, pages 33G-43G; D.R. ILLINGWORTH et al: "Hypolipidemic effects of HMG-CoA reductase inhibitors in patients with hypercholesterolemia" * Whole article * | 1-3 | A 61 K 31/215 // (A 61 K 31/215 A 61 K 31:19 ) |
| Y | IDEM | 4-10 | |
| X | THE NEW ENGLAND JOURNAL OF MEDICINE, vol. 319, no. 1, 7th July 1988, pages 24-33, The Massachusetts Medical Society; S.M. GRUNDY et al.: "HMG-CoA reductase inhibitors for treatment of hypercholesterolemia" * Whole article * | 1-3 | |
| Y,D | DRUGS, vol. 36, supplement 3, 1988, pages 63-71, ADIS Press Ltd; D.R. ILLINGWORTH: "An overview of lipid-lowering drugs" * Whole article * | 4-10 | |
| Y,D | METABOLISM, vol. 39, no. 4, April 1990, pages 403-409, W.B. Saunders Co. Harcourt Brace Jovanovich, Inc.; D.R. ILLINGWORTH et al.: "The hypolipidemic effects of lovastatin and clofibrate alone and in combination in patients with type III hyperlipoproteinemia" * Whole article * | 4-10 | TECHNICAL FIELDS SEARCHED (Int. Cl.5) A 61 K |
| A | THE JOURNAL OF JAPAN ATHEROSCLEROSIS SOCIETY, vol. 16, no. 1, 1988, pages 5-8; G. YOSHINO et al.: "Effect of CS-514 on type III and type III-like hyperlipoproteinemia" * Whole article * | 1-3 | |
| Y | EP-A-0 373 507   (E.R. SQUIBB & SONS, INC.) * Page 2; page 3, lines 1-5 * | 4-10 | |

$-/-$

The present search report has been drawn up for all claims

| Place of search | Date of completion of search | Examiner |
|---|---|---|
| The Hague | 04 December 91 | GAC G. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

& : member of the same patent family, corresponding document

**European Patent Office**

# EUROPEAN SEARCH REPORT

**EP 91 11 4074**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | $-\ -\ -\ -\ -$ | 1-3 | |

TECHNICAL FIELDS SEARCHED (Int. Cl.5)

The present search report has been drawn up for all claims

| Place of search | Date of completion of search | Examiner |
|---|---|---|
| The Hague | 04 December 91 | GAC G. |